# EUROPEAN PATENT APPLICATION

(11) **EP 1 462 528 A1**
(43) Date of publication of application: **29.09.2004**
(21) Application number: 04251254.1
(22) Date of filing: 04.03.2004
(51) Int. Cl.: C12Q 1/68, C07H 21/00

(54) **Nucleic acid immobilization and use as a biosensor**

(30) Priority: 26.03.2003 JP 2003086362
(71) Applicant: SEIKO EPSON CORPORATION, Shinjuku-ku, Tokyo 163-0811 (JP)
(72) Inventor: Takiguchi, Hiroshi, Suwa-shi, Nagano-ken (JP); Fukushima, Hitoshi, Suwa-shi, Nagano-ken (JP)
(74) Representative: Sturt, Clifford Mark

(57) **Abstract**

A superior adsorption method for immobilizing nucleic acid probe on the surface of a solid phase substrate is provided. A nucleic acid immobilization method for immobilizing nucleic acid on a solid phase substrate comprising: bringing the above-mentioned solid phase substrate into contact with a composition comprising a total concentration of 0.1 to 2 µM of a nucleic acid as a probe and a compound or a salt thereof, the compound being represented by the following formula:

**HS―L**^{**1**}**―L**^{**2**}**―R** (I)

wherein L¹ is a single bond or a C₁₋₁₅ alkylene group; L² is a single bond, nucleic acid, a polyethylene glycol group, -CO-NH-, or -NH-CO-; R is a hydroxyl group, an amino group, a ferrocenyl group, or a carboxyl group; provided that neither L¹ nor L² is a single bond, and incubating the composition in contact with a surface of the solid phase substrate.

## Description

### BACKGROUND OF THE INVENTION

### 1. Field of the invention

The present invention relates to a nucleic acid immobilization method, and to a biosensor manufacturing method and a nucleic acid detection method using same.

### 2. Description of the Related Art

Biosensors, such as microarray technology using nucleic acid probes, have recently come into widespread use as means for detecting target nucleic acid molecules, analyzing sequences, and gene mapping. The hybridization method, which is a representative example, is a method for capturing the target nucleic acid molecules with a nucleic acid probe by utilizing the interaction of complementary nucleic acid strands (hybridization), and determining directly or indirectly the presence of the target nucleic acid molecule (Example: detection by the emission intensity of fluorescent molecules adhering to the sample) to analyze all or part of the nucleic acid sequence of the target nucleic acid,. A single strand nucleic acid molecule having a sequence of all or part of the target nucleic acid molecule is commonly used as the probe, and the solid support for detection of the nucleic acid molecule is formed by immobilizing (for example, by a covalent bond, ionic bond, adsorption, or biological specific binding) the probe on a solid phase substrate. Consequently, according to stoichiometry, the higher the density of the nucleic acid immobilized on the solid phase substrate, the more the amount of complementary nucleic acid that can be captured by hybridization. Nonetheless, there is the concern that making the distance between nucleic acids too narrow may inhibit hybridization. It is, therefore, necessary to adjust the density and interval of the nucleic acid probes on the surface of the solid phase substrate to the optimal level in order to efficiently capture nucleic acid molecules at maximum amount.

The major methods used to immobilize nucleic acid on a solid phase substrate are: (1) a method comprising the step of carrying out a nucleic acid extension reaction on the surface of the substrate, i.e., directly synthesizing a nucleic acid probe on the substrate; and (2) a method comprising the step of pre-synthesizing the nucleic acid probe before conducting the nucleic acid immobilization step, and then immobilizing the probe on the surface of the substrate.

Examples of (1) above include: (1a) photolithography technology comprising repeating the steps of immobilizing a support having a linker with a protecting group onto the substrate, applying light to the linker to remove the protecting group while masking, reacting the deprotected linker with a nucleic acid monomer having a protecting group to form a bond; (1b) a two-stage masking technique; and (1c) a technology that synthesizes the objective nucleic acid probe by directly applying light to the reaction site by reflecting the light with a mirror surface without masking. Examples of (2) above include the adsorption method that causes adsorption of a nucleic acid molecule to the substrate by bonding an adsorptive group at an end of the nucleic acid by a linker.

When using the above-mentioned adsorption method, relatively low molecular weight molecules, called spacer molecules, are generally inserted between nucleic acid probes. Low molecular weight molecules that have no effect on the operation of the biosensor are commonly used for the above-mentioned spacer molecules, and appear to have an important role in optimizing density of nucleic acid probes and the interval of probes. It is known that some alkanethiol compounds have a thiol group on the end that can be used to form a self assembled monolayer (SAMs), and that when the surface of the substrate is formed by gold, SAM formation occurs based on gold-sulfur bond immobilizing thiol groups. For example, it has been reported that in a test of the adsorption of single-stranded DNA having a thiolated end, 6-mercapto-1-hexanol was used as the spacer molecule (For example, refer to Tonya M. Herne et al., Journal of American Chemical Society, 119, pp. 8916-8920 (1997).).

Well-known adsorption methods include embedding, co-adsorption, and substitution. Embedding method is a film formation method comprising the steps of carrying out a high-density adsorption of a nucleic acid probe onto a solid phase substrate, and embedding spacer molecules in the remaining spaces. The substitution method is a method to form a film by substituting adsorbed spacer molecules with a nucleic acid probe.

Nonetheless, Embedding method has the problem that it is not possible to completely control the density of the nucleic acid probe during the initial step of adsorbing the nucleic acid probe. There have been reports (For example, refer to Tonya M. Herne et al., Journal of American Chemical Society, 119, pp. 8916-8920 (1997).) that controlling the time of the adsorption of the nucleic acid probe is means to resolve this problem, but it is extremely difficult to efficiently control nucleic acid density using an exceedingly unstable parameter such as reaction time. In addition, there have been reports (For example, refer to A. B. Steel et al., Biophysical Journal, Vol. 79, pp. 975-981 (2000).) that the step of embedding the spacer molecules changes the density of the nucleic acid probe that was first adsorbed, thus making this is an unsuitable method to adjust density. Further, substitution is impractical because the film formation time takes too long. Meanwhile, co-adsorption is a method in which a solid phase substrate is immersed in a mixed solution of multiple types of molecules including the nucleic acid probe, and the amount of molecule adsorption in relation to the solid phase substrate is adjusted by varying the mixture percentages of the above-mentioned molecules. Theoretically, the density of the nucleic acid probe in the biosensor can be regulated just by initially adjusting the compositional percentages of the mixed solution.

### SUMMARY OF THE INVENTION

There is a problem that the adsorption speed of spacer molecules such as 6-mercapto-1-hexanol is extremely rapid compared to that of the nucleic acid molecules, and obtaining adsorption at an optimal density is difficult unless there is a suitably high concentration of nucleic acid molecules in the mixed solution. Moreover, the relative adsorption speeds of the two are prone to change depending on the structure of the nucleic acid probe, which makes it all the more difficult and complicated to adjust the density of the nucleic acid. Specifically, an object of the present invention is to provide an effective and superior co-adsorption method in order to resolve these problems and to allow adsorption of a nucleic acid probe on the surface of a solid phase substrate at the optimal density.

As a result of concerted research, the inventors completed the present invention by discovering that when conducting co-adsorption using a composition comprising a total concentration of 0.1 to 2 µM of a nucleic acid probe molecules and spacer molecules or salts thereof represented by the following formula:

HS-L¹-L²-R (I)

wherein L¹ is a single bond or a C₁₋₁₅ alkylene group, L² is a single bond, a nucleic acid, a polyethylene glycol group, -CO-NH-, or -NH-CO-, and R is a hydroxyl group, an amino group, a ferrocenyl group, or a carboxyl group; provided that neither L¹ nor L² is a single bond (abbreviated as "compound I" below), not only was it unexpectedly possible to immobilize nucleic acid probe at an very satisfactory density, but it was also extremely easy to finely adjust the composition percentage of the nucleic acid probe in the composition, and as a result, it was extremely easy to adjust the immobilization density of the nucleic probe without depending on the combination of the probe and the spacer molecule.

Specifically, the present invention relates to: [1] A method for immobilizing nucleic acid on a solid phase substrate comprising: bringing the above-mentioned solid phase substrate in contact with a composition comprising a total concentration of 0.1 to 2 µM of a nucleic acid as a probe and a compound or a salt thereof represented by the following formula:

HS-L¹-L²-R (I)

wherein L¹ is a single bond or a C₁₋₁₅ alkylene group; L² is a single bond, a nucleic acid, a polyethylene glycol group, -CO-NH-, or -NH-CO-; R is a hydroxyl group, an amino group, a ferrocenyl group, or a carboxyl group; provided that neither L¹ nor L² is a single bond, and incubating the composition in contact with a surface of the solid phase substrate; [2] The method according to [1], wherein the nucleic acid as a probe comprises a polynucleotide or an oligonucleotide consisting of modified or unmodified, single-stranded DNA, RNA, PNA, CNA, or HNA; [3] The method according to [1], wherein the nucleic acid as the probe comprises at the 3' end or the 5' end a group represented by the following formula:

HS-L³-L⁴- (II)

wherein L³ is a C₁₋₁₅ alkylene group, and L⁴ is a single bond or a spacer; [4] The method according to [1], wherein the nucleic acid as the probe has at the 5' end a group represented by the following formula: 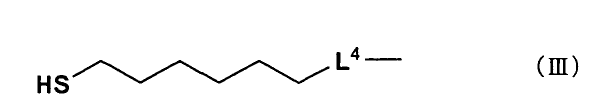 wherein L⁴ is a single bond or a spacer; [5] The method according to [4], wherein L⁴ is a nucleic acid, -CO-NH-, -NH-CO-, a polyethylene glycol group, or a polyethylene glycol phosphate group; [6] The method according to [1], wherein the total concentration of the nucleic acid and the compound I or the salt thereof in the composition is 0.5 to 1.5 µM; [7] The method according to [1], wherein the total concentration of the nucleic acid and the compound I or the salt thereof in the composition is 1 µM; [8] The method according to [1], wherein the composition comprises the nucleic acid and the compound I at a ratio of 40/60 to 60/40; [9] The method according to [1], wherein R in the formula I is a hydroxyl group; [10] The method according to [1], wherein L¹ in the formula I is a single bond, and L² is a polyethylene glycol group; [11] The method according to [1], wherein L¹ in the formula I is a C₄₋₈ alkylene group, and L² is a single bond; [12] The method according to [1], wherein the compound I is 6-mercapto-1-hexanol; [13] The method according to [1], wherein the solid phase substrate is a single layered substrate or a multiple layered substrate comprising at least one material selected from the group consisting of glass, polymer resin and metal; [14] The method according to [1], wherein the surface of the solid phase substrate to which nucleic acid is adsorbed is coated with a thin gold film; [15] The method according to [1], wherein the solid phase substrate is a glass substrate with a thin gold film vapor-deposited on its surface, and may further comprises, at least one intermediate layer between the thin gold film and the glass substrate; [16] The method according to [1], wherein the nucleic acid as the probe has 15 to 30 base length; [17] The method according to [1], wherein the incubation is carried out at a temperature of 25° C to 40° C; [18] The method according to [1], wherein the nucleic acid as the probe is a polynucleotide or an oligonucleotide consisting of single-stranded DNA, RNA, or PNA, and may also has the group represented by formula II, the compound I is 6-mercapto-1-hexanol; the total concentration of the nucleic acid and 6-mercapto-1-hexanol in the composition is 0.5 to 1.5 (µM; and the solid phase substrate is a glass substrate with a thin gold film vapor-deposited on its surface, and further, at least one intermediate layer may be made between the thin gold film and the glass substrate; [19] A method for manufacturing a biosensor having a nucleic acid probe as a sensing site comprising the use of the nucleic acid immobilization method according to any of [1] to [18]; [20] A biosensor comprising a solid phase substrate and a nucleic acid probe thereon as a sensing site, the biosensor manufactured by a method comprising the steps of:bringing a composition containing a nucleic acid and a compound or a salt thereof at a total concentration of 0.5 to 1.5 µM into contact with the solid phase substrate, the compound being represented by the following formula;

HS-L¹-L²-R (I)

wherein L¹ is a single bond or a C₁₋₁₅ alkylene group; L² is a single bond, a nucleic acid, a polyethylene glycol group, - CO-NH-, or -NH-CO-; R is a hydroxyl group, an amino group, a ferrocenyl group, or a carboxyl group; provided that neither L¹ nor L² is a single bond, and incubating the composition in contact with a surface of a solid phase substrate;[21] The biosensor according to [20], wherein the compound I is 6-mercapto-1-hexanol, the solid phase substrate is a glass substrate with a thin gold film vapor-deposited on its surface, and further, the solid phase substrate may comprises at least one intermediate layer between the thin gold film and the glass substrate; [22] A method for detecting a target nucleic acid molecule in a test sample by detecting a measurable signal, comprising the steps of: (a) bringing the test sample into contact with a biosensor having a nucleic acid probe manufactured by using the nucleic acid immobilization method according to claim 1, and incubating the test sample in contact with a surface of the biosensor; (b) applying light to the solid phase substrate of the biosensor from the opposite side of the surface to which nucleic acid is immobilized, ontinuously or intermittently from before to after step (a); and (c) measuring a shift of reflectivity of the solid phase substrate by detecting the reflection of the light applied in step (b); [23] The method according to [22], wherein the method is used for detecting DNA, RNA, or single nucleotide polymorphisms; [24] A use of a composition comprising a nucleic acid and compound I or salt thereof at a total concentration of 0.5 to 1.5 µM, for manufacturing a biosensor having a nucleic acid probe as a sensing site; [25] A use of a nucleic acid and compound I or salt thereof, for manufacturing the composition for the use according to [24].

The nucleic acid molecule and the spacer molecule or the salt thereof used in the "nucleic acid immobilization method" of the present invention are used after pre-mixing and forming a composition prior to application in an adsorption reaction with the surface of the solid phase substrate, and, ordinarily, this composition is used as a solution. The composition used in the present invention ordinarily comprises the nucleic acid and the spacer molecule or the salt thereof at a total concentration of 0.1 to 5 µM, and the surface of the solid phase substrate is preferably covered with the nucleic acid probe as far as the total concentration is limited to that range. Within this range, a preferable total concentration is 0.1 to 2 µM, better is 0.5 to 1.5 µM, even better is 0.5 µM to approximately 1 µM, and optimum is approximately 1 µM. Specifically, in the nucleic acid immobilization method of the present invention, the optimal adsorption of the nucleic acid probe and spacer molecules is obtained when approximately a total concentration of 1 µM is used.

The ratio (mol%) of the nucleic acid molecules and the spacer molecules and the salts thereof in the above-mentioned mixed solution is not particularly limited, and can be suitably selected by the practitioner depending on the nucleic acid density on the objective biosensor, the structure of the nucleic acid probe, the spacer molecule used, and the type of solid phase substrate used. Implementation is possible with any composition ratio depending on the objective if the mixed solution has a ratio (mol%) of nucleic acid molecules and spacer molecules in the range of approximately 1/ 99 to approximately 99/ 1. According to the nucleic acid immobilization method of the present invention, a high concentration of nucleic acid molecules as used in prior art is unnecessary, and a nucleic acid molecule and spacer molecule composition of roughly 50/50 (mol% ratio) can achieve the most efficient adsorption of nucleic acid molecules. Naturally, it is possible to cover the surface of the solid phase substrate by finely adjusting the mixture and composition in any range of nucleic acid molecules/ spacer molecules= approximately 1/99 to approximately 10/90, approximately 10/90 to approximately 20/80, approximately 20/80 to approximately 30/70, approximately 30/70 to approximately 40/60, approximately 40/60 to approximately 50/50, approximately 50/50 to approximately 60/40, approximately 60/40 to approximately 70/30, approximately 70/30 to approximately 80/20, approximately 80/20 to approximately 90/10, and approximately 90/10 to 99/1 (the above are mol% concentration ratios).

The respective methods of preparing the nucleic acid probe solution and the solution and the spacer molecule solution used in the present invention, as well as the method of mixing both solutions, are not particularly limited, and as long as both are dissolved and the adsorption reaction to the surface of the solid phase substrate is not inhibited, any solvent or mixture method may be used. For example, if a total concentration of X µM (X is 0.1 to 5) of nucleic acid probe and spacer molecules is desired in the mixed solution, a mixed solution of the targeted X µM may be prepared by preparing the nucleic acid probe solution and spacer molecule solution separately to their respective X µM concentrations, and mixing both in a suitable target ratio. The following may be cited as suitable solvents for use in preparing the above-mentioned nucleic acid probe and spacer molecule solutions: various types of phosphate buffer solution (for example, PBS (50 mM KPO₄, 5 mM EDTA, 1 M NaCl, pH7.0), etc.), and of TE buffer solution (mixed buffer solution of Tris-HCl and EDTA, pH 8.0). The pH of the applicable buffer solution is not particularly limited, and ordinarily may be in the vicinity of pH 5.5 to 8.5, preferably in the vicinity of pH 7 to 8. Here, it is possible to conduct suitable high density immobilization by including a salt such as NaCl (preferably, NaCl) in the above-mentioned buffer solution.

The "salt" used in the present invention is not particularly limited if it can form a salt with the spacer molecule, and may be any salt form. Specifically, the following may be cited: inorganic added salts (for example, hydrochloride salt, sulfate salt, carbonate salt, bicarbonate salt, hydrogen bromide salt, hydrogen iodide salt, etc.); organic carboxylic acid added salts (for example, acetate salt, maleate salt, lactate salt, tartrate salt, trifluoroacetate salt, etc.); organic sulfonic acid added salts (for example, methane sulfonate salt, hydroxymethane sulfonate salt, hydroxyethane sulfonate salt, benzene sulfonate salt, toluene sulfonate salt, taurine salt, etc.); amine added salts (for example, trimethylamine salt, triethylamine salt, pyridine salt, procaine salt, picoline salt, dicyclohexylamine salt, N,N'-dibenzylethylenediamine salt, N-methylglucamine salt, diethanolamine salt, triethanolamine salt, tris(hydroxymethylamino)methane salt, fenetylbenzylamine salt, etc.); amino acid added salts (for example, arginine salt, lysine salt, serine salt, glycine salt, asparagine salt, glutamate salt, etc.).

The "nucleic acid" in the nucleic acid immobilization method of the present invention means a single- or doublestranded oligonucleotide or a polynucleotide, part or all of which may be modified (including substitutions); preferably, a single-stranded oligonucleotide or polynucleotide, part or all of which may be modified (including substitutions). Suitable examples of the "nucleic acid" may be selected from group consisting of DNA, RNA, PNA (peptide nucleic acid), CNA (amino cyclohexanyl nucleic acid), HNA (hexitol nucleic acid), p-RNA (pyranosyl RNA), oligonucleotides consisting of the above-mentioned nucleic acid molecules, and polynucleotides comprising the above-mentioned nucleic acid molecules, etc. SNPs (single nucleotide polymorphisms) are also included in the above-mentioned nucleic acids.

More over, the "nucleic acid" means a molecule having a group at the end of the molecule suitable for adsorption to the surface of a solid phase substrate, and is not particularly limited as long as the group is suitable for adsorption to the substrate. Preferable example include a group that bonds to the 3' end or 5' end represented by the following formula:

HS-L³-L⁴- (II)

wherein L³ is a C₁₋₁₅ alkylene group, and L⁴ is a single bond or a spacer. The C₁₋₁₅ alkylene group of the above-mentioned L³ means an alkylene group with 1 to 15 carbons (specifically, -CH₂-, - (CH₂)₂-, - (CH₂)₃-, - (CH₂)₄-, - (CH₂)₅-, - (CH₂)₆-, - (CH₂)₇-, -(CH₂)₈-, - (CH₂)₉-, - (CH₂)₁₀-, - (CH₂)₁₁-, - (CH₂)₁₂-, - (CH₂)₁₃-, -(CH₂)₁₄-, -(CH₂)₁₅-). These are not particularly limited, but a C₄₋₁₁ alkylene group is preferable, a C₄₋₈ alkylene group is more preferable, and a C6 alkylene group represented by -(CH₂)₆- is most preferable. The above-mentioned L⁴ may be either a single bond or a spacer, but a single bond, a group comprising polyethylene glycol (for example, a polyethylene glycol group, a polyethylene glycol phosphate group, etc.), nucleic acid, -CO-NH-, and -NH-C0- is preferable. More preferable is a group expressed by the following formula: 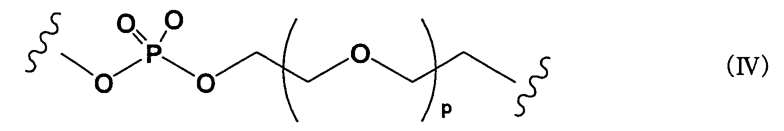 wherein p is an integer of 1 to 10.

A preferable example of the group represented by formula (II) is when L³ is a C₄₋₈ alkylene group, and L⁴ is a single bond or a group represented by formula (IV), and a more preferable example is when L³ is -(CH₂)₆-, and L⁴ is a single bond or a group represented by formula (IV) wherein p is the integer 6.

The length of the base comprising the nucleic acid probe used in the present invention is not particularly limited as long as it corresponds to the objective, but a nucleic acid probe having nucleic acid with 4 to 50 bases is preferable, 10 to 35 bases is more preferable, and even more preferable is a nucleic acid probe having nucleic acid with 10 to 30 bases.

The "nucleic acid probe" used in the present invention has the same meaning as the above-mentioned "nucleic acid".

The spacer molecule used in the present invention may be dissoluble into a solution with the nucleic acid molecules, and is not particularly limited as long as this spacer molecule is a low molecular weight substance that does not inhibit the function of the nucleic acid probe immobilized on the surface of the solid phase substrate and has no effect on the operation as a biosensor. The practitioner may suitably select the spacer molecule corresponding to the density of the nucleic acid on the targeted biosensor, the structure of the nucleic acid probe, the type of spacer molecule to be used, and the type of solid phase substrate to be used. Preferable examples include low molecular weight compounds having a thiol group on the end, and more preferably, an alkanethiol compound represented by the following formula:

HS-L¹-L²-R (I)

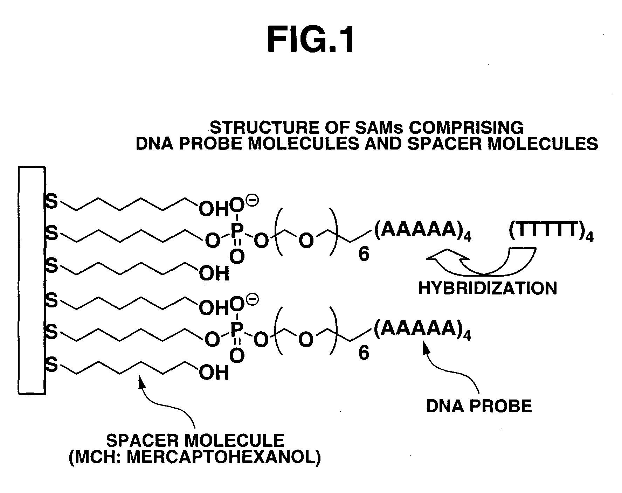

wherein L¹ is a single bond or a C₁₋₁₅ alkylene group, L² is a nucleic acid, a polyethylene glycol group, -CO-NH-, or -NH-CO-, R is a hydroxyl group, an amino group, a ferrocenyl group, or a carboxyl group; however, L¹ and L² may not both be single bonds. Preferable examples of the above-mentioned Compound I include hydroxyalkanethiol compounds in which R is a hydroxyl group; more preferably, hydroxyalkanethiol compounds in which L¹ is a C₄₋₁₁ alkylene group, L² is a single bond, and R is a hydroxyl group; and even more preferably, 6-mercapto-1-hexanol represented by HS-(CH)₆-OH. Figure 1, which will be described later, indicates a schematic diagram of the surface of a solid phase substrate formed by subjecting a nucleic acid probe and spacer molecules to a co-adsorption reaction.

The "solid phase substrate" used in the present invention is not particularly limited as long as the substrate does not prevent the immobilization of nucleic acid, and any kind of solid phase substrate may be used. The practitioner may suitably select the optimal conditions for the material, the number of layers, and the types and thicknesses of the "solid phase substrate" depending on the type of adsorption group presented by the nucleic acid molecules to be immobilized, and on the signal detection means adopted in order to detect the target nucleic acid molecule. Preferable substrates may be selected from glass substrate, metal substrate (for example, gold, silver, copper, aluminum, platinum, aluminum oxide, SrTiO₃, LaAlO₃, NdGaO₃, and ZrO₂, etc.), silicon substrate (for example, silica oxide) and polymer resin substrate (for example, polyethlene terephthalate, polycarbonate), etc.

The solid phase substrate used in the present invention may be a substrate comprising a single material of those listed above, may comprise a thin film (first layer) on the surface of the substrate (first substrate) consisting of at least one material other than the material of the first substrate, or there may be at least one intermediary layer (second layer, third layer, etc.) between the above-mentioned first substrate and the above-mentioned first layer. Specific examples of a preferable "solid phase substrate" include the above-mentioned glass substrate having a metal film (preferably, gold thin film, silver thin film, copper thin film, platinum thin film) as the above-mentioned first layer. Intermediate layers comprising other materials may be made between the above-mentioned glass substrate and the above-mentioned metal film.

The formation of the various metal films (preferably, gold thin film, silver thin film, copper thin film, platinum thin film), especially of the above-mentioned first layer, may be made by self-evident, well-known methods or methods based on these. For example, the thin films may be made by electroplating, non-electric plating, sputtering, vapor deposition, or ion plating, etc. If the surface of the above-mentioned metal film is contaminated (for example, by organic substances in the atmosphere), ordinarily the surface of the metal is washed with an organic solvent, and as necessary, the contamination is removed using methods such as decomposition by washing with a strong acid, or decomposition by ozone, etc. produced by ultraviolet rays. Preferable examples include boiling and washing the metal surface with an organic solvent (for example, acetone, etc.), followed by washing the metal with a UV ozone washer, or by washing using piranha solution (mixed solution of hydrogen peroxide/concentrated sulfuric acid = 30/70).

The thickness of the "solid phase substrate" used in the present invention is not particularly limited, but, ordinarily, the above-mentioned first substrate is about 0.1 to 30 mm, preferably, about 0.1 mm to 20 mm. The above-mentioned second and third layers etc. are ordinarily 1 to 2000 nm, preferably, 1 to 1000 nm, and more preferably, 100 to 500 nm.

In the "nucleic acid immobilization method" of the present invention, the reaction temperature when bringing the mixed solution of nucleic acid and spacer molecules into contact with the solid phase substrate and incubating is not particularly limited, but ordinarily, is in the vicinity of 0 to 40° C, preferably in the vicinity of 20 to 35° C. The reaction time is not particularly limited, but ordinarily, incubation of 30 minutes to 24 hours is sufficient, preferably, 1 to 12 hours.

The present invention also provides a biosensor manufacturing method comprising the use of the above-mentioned "nucleic acid immobilization method", and a nucleic acid molecule detection method comprising the use of the above-mentioned "nucleic acid immobilization method", the various types of conditions used in the related manufacturing method and nucleic acid molecule detection method are the same as in the above-mentioned definitions.

The biosensor manufactured by the biosensor manufacturing method related to the present invention is a biosensor having at least 1 x 10² to 1 x 10¹⁶ nucleic acid molecules per cm², and preferably, at least 4 x 10² to 1 x 10¹⁵ nucleic acid molecules.

The "nucleic acid molecule detection method" related to the present invention means using a biosensor obtained by the nucleic acid immobilization method or manufacturing method related to the present invention, and determining the present or absence of the related target molecule by capturing the target nucleic acid molecule or its analog contained in a test sample. This may be a determination method that also comprises a step of analyzing the sequence of the target nucleic acid molecule. Specifically, this detection method is used to detect oligonucleotides and polynucleotides of DNA (including cDNA), oligonucleotides and polynucleotides of RNA (mRNA, etc.), and single nucleotide polymorphisms (SNPs) of these nucleic acids, etc. The above-mentioned method " to capture the target nucleic acid molecule or its analog" is not particularly limited, but a preferable example is hybridization.

Means using surface plasmon resonance (SPR), or means that labels the target nucleic acid molecules, etc. with fluorescent molecules and detects changes in the emission intensity may be cited as examples of the "method to detect target nucleic acid molecules in a test sample by detecting measurable signals" of the present invention, or the preferable means in order "to detect measurable signals" based on the use of the "nucleic acid immobilization method" or "biosensor manufacturing method" related to the present invention. This means is not particularly limited, but a more preferable example is means using surface plasmon resonance (SPR). The target nucleic acid molecule can be efficiently detected by the nucleic acid molecule detection method related to the present invention.

Surface plasmon resonance (SPR) is means to analyze test samples by irradiating light at a critical angle or more from the opposite site of the surface of a solid phase substrate on which nucleic acid is immobilized, by detecting the changes of the intensity of that reflected light, and by calculating the reflectivity of the immobilized nucleic acid surface from the attenuation. When using surface plasmon resonance (SPR) as the signal detection means of the present invention, an optical system using optical fibers, prisms, diffraction lattice, and a light guide, etc. may be used as the method to implement surface plasmon resonance, and glass, primer resin or plastic, etc. may be used as the material, specifically, as the solid phase substrate material of the biosensor. The object measured by surface plasmon resonance may be the wavelength of the light or the incidence and reflection angles; an LED, LD, or white light, etc. may be used as the light source; and a CCD, PD, or optical position sensor, etc. may be used as the detector.

Preferable examples of the above-mentioned fluorescent molecules when using a detection method by labeling target nucleic acid molecules with fluorescent molecules include FITC (fluorescein isothiocyanate), RITC (rhodamine isothiocyanate), TMRITC (tetramethyl rhodamine isothiocyanate), Cy3 (carboxymethyl indocyanin), and PE (phycoerythrin). Here, because discoloration occurs with FITC and PE even when using color preservative after the reaction, it is preferable to keep a low temperature by using a shade (for example shading with aluminum foil, etc.). In addition, quantum dot substance (Warren C.W. Chan and Shunming Nie, Quantum Dot Bioconjugates for Ultrasensitive Nonisotopic Detection, Science 281, 2016 (1998)) may be used as the fluorescent substance for labeling. Quantum dots are substances that have a semiconductor nano-area of several nm to several hundred nm and that take the behavior of a single electron as the operational principle. Preferable examples of the above-mentioned quantum dot substances include, for example, substances comprising microparticles of compound semiconductors such as ZnS, and CdSe, etc. These substances emit fluorescent light when excited by UV lasers, and the wavelengths vary depending on the particle size, but because the excitation wavelength is constant, it is possible for a single laser excitation to cause fluorescent coloration of various colors.

The nucleic acid probe used in the present invention may be acquired as a commercial product or may be easily manufactured by self-evident, well-known methods or methods based on these. For example, when acquiring a commercial product, normally, individual powders may be purchased. In addition, spacer molecules used in the present invention may also be acquired as a commercial product, or may be easily manufactured by self-evident, well-known methods or methods based on these.

The meanings of the terms used in the SPECIFICATION of the present application as well as in the BACKGROUND OF THE INVENTION are as above, but the characteristics of the present invention will be described below in order to make it even easier to understand the present invention. First characteristic of the present invention is that, in a method to immobilize nucleic acid on the surface of a solid phase substrate, a composition comprising a nucleic acid and a spacer molecule at a total concentration of approximately 0.1 to 5 µM, preferably at a total concentration of approximately 0.1 to 2 µM, and optimally at a total concentration of approximately 1 µM, is brought into contact with the above-mentioned solid phase substrate. A preferable example of the above-mentioned spacer molecule is an alkanethiol compound or salt thereof represented by the following formula:

HS-L^{1a}-OH

wherein L^{1a} is a C₄₋₈ alkylene group; and optimally, 6-mercapto-1-hexanol. Preferable as a composition comprising a nucleic acid and spacer molecules is various types of phosphate buffer solution (in the vicinity of pH 5.5 to 8.5, preferably pH 7). The optimal combination of a solid phase substrate is a glass substrate with vapor deposition of a thin gold film on the surface thereof.

Second characteristic of the present invention is a biosensor manufacturing method comprising the use of the above-mentioned "nucleic acid immobilization method" of the present invention. The biosensor manufactured by related the manufacturing method is a biosensor on which a nucleic acid probe is immobilized, and the density of the above-mentioned nucleic acid can be easily and finely adjusted in a range from high to low.

Further, third characteristic of the present invention is a method to detect labeled nucleic acid molecules comprising the use of a biosensor obtained by a manufacturing method comprising the use of the above-mentioned "nucleic acid immobilization method" or the "biosensor manufacturing method" of the present invention. To carry out the detection method, the superior biosensor may be used as previously described, but, for example, even more highly precise and efficient detection of target nucleic acid molecules is possible by capturing and detecting the presence of the target nucleic acid using surface plasmon resonance (SPR). In particular, by using the above-mentioned surface plasmon resonance (SPR) it is possible to continuously ascertain the capture of the target nucleic acid molecules by the biosensor over time.

The present invention can provide a superior nucleic acid immobilization method, a biosensor manufacturing method comprising the use of the related immobilization method, and a target nucleic acid molecule detection method comprising the use of the related immobilization method. According to the nucleic acid immobilization method related to the present invention, not only is it possible to immobilize the nucleic acid probe at the optimal density in merely one step, but causing the nucleic acid probe to start up from the probe, preventing the adsorption of contamination on the surface of the substrate, and providing electrochemical insulating characteristics, etc. are also possible. According to this method, it is extremely easy to adjust the density of the biosensor just by adjusting the mixture concentration of the nucleic acid probe and the spacer molecule prior to the immobilization reaction.

### BRIEF DESCRIPTION OF THE DRAWINGS

Fig. 1 A figure indicating a solid phase substrate surface to which a nucleic acid probe and 6-mercapto-1-hexanol are co-adsorbed;
Fig. 2 A figure indicating the results of using surface plasmon resonance (SPR) to verify the co-adsorption process of nucleic acid probe to the surface of a solid phase substrate;
Fig. 3 A figure indicating the results of using surface plasmon resonance (SPR) to verify co-adsorption to a solid phase substrate and hybridization when using a total concentration of 10 µM of nucleic acid probe and 6-mercapto-1-hexanol in different composition ratios;
Fig. 4 A figure indicating the results of using surface plasmon resonance (SPR) to verify the co-adsorption to a solid phase substrate and hybridization when using a total concentration of 1 µM of nucleic acid probe and 6-mercapto-1-hexanol in different composition ratios;
Fig. 5 A figure indicating the results of using SPR to evaluate the effects of changes in the total concentration of nucleic acid probe and 6-mercapto-1-hexanol on the hybridization reaction; and
Fig. 6 Figures indicating the results of using QMC measurements to evaluate the concentration dependence of simple film formation of 6-mercapto-1-hexanol(A) and nucleic acid probe (B) .

### DESCRIPTION OF THE PREFERRED EMBODIMENTS

The reference examples, examples and experimental examples of the present invention described below are representative, and the present invention is not limited to the specific examples below. The practitioner may implement the present invention to the maximum by variously modifying the examples described below, and the related modifications will fall within the scope of the patent claims of the present application.

### Example 1

Preparation of a mixed solution and a solid phase substrate in order to immobilize a nucleic acid probe
(1) Nucleic acid probe: A nucleic acid probe represented by the formula (dA)₂₀-(CH₂)-O-CH₂)ₙ-PO₄-(CH₂)₆-SH was purchased as the nucleic acid probe to provide for adsorption to the solid phase substrate. Here, dA means deoxyadenosine 5'-phosphate.
(2) Spacer molecule: 6-mercapto-1-hexanol (MCH) was purchased for use as the spacer molecule.
(3) Preparation of buffer solution: PBS buffer solution (50 mM KPO₄, 5 mM EDTA, 1M NaCl, pH 7.0) was prepared. The nucleic acid probe and spacer molecule were separately dissolved in the above-mentioned PBS solution, and 2 types, 1 µM and 10 µM, were used respectively.
(4) Preparation of solid phase substrate: A glass substrate with a thin gold film on the surface thereof was prepared according to the above-mentioned manufacturing method, and was used as the solid phase substrate.

### Example 2

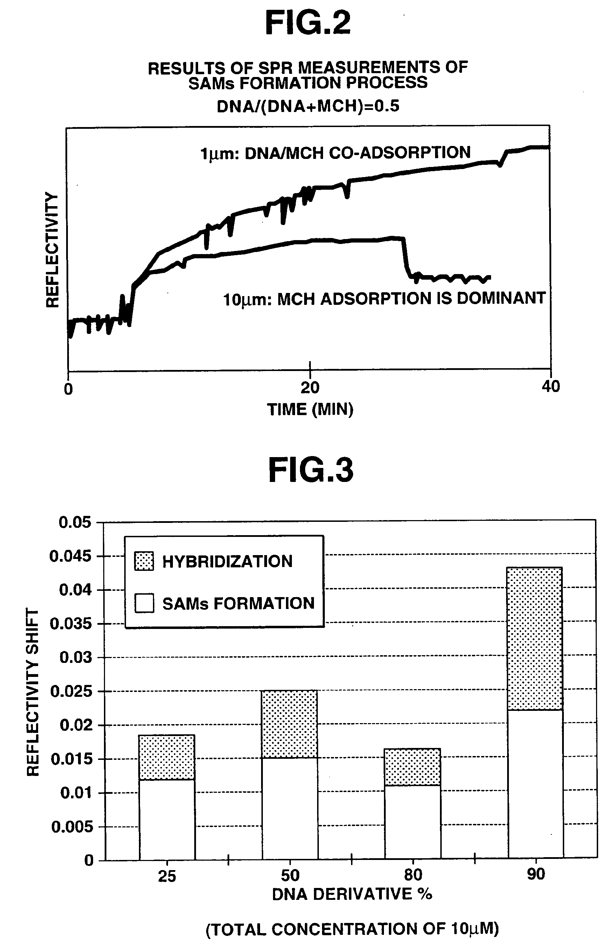

Co-adsorption of nucleic acid probe and MCH
(1) Co-adsorption: Mixed solutions were prepared by mixing the nucleic acid probe solution and spacer molecule solution. 1 µM nucleic acid probe solution is mixed with 1 µM spacer molecule solution, and 10 µM probe solution is mixed with 10 µM spacer molecule solution, at a ratio of 25/75, 50/50, 75/20 (mol%) respectively. The solid phase substrate with its surfaces cleaned was immersed in the above-mentioned mixed solutions, and incubated for 1 to 3 hours. The intensity of the reflected light was measured using SPR equipment at a fixed observation angle.
(2) Results:
   Indicated in Figure 2 are the results of evaluating the process of the nucleic acid probe adsorption by the amount of shift of reflected light measured by SPR. A change of the intensity of the reflected light is related to a shift of reflectivity and the increase or decrease of the film thickness. The co-adsorption at a total concentration of 10 µM caused extremely large initial shift of the reflectivity. The reflectivity does not, however, shift very much after the initial shift, and a considerable portion of adsorbed molecules are washed away from the surface when rinsing. The reflectivity after rinsing was about the same strength as that of the inflection point observed at the beginning of adsorption, and therefore, which indicates that film formation under these reaction conditions was completed in about one minute, and it appears that 6-mercapto-1-hexanol is superior in adsorption to a nucleic acid probe molecule. It appears that the rinsing operation removed most of the nucleic acid probe.
   On the other hand, in co-adsorption at the low concentration of 1 µM, the increase of reflectivity of the adsorption process was gradual, and adsorption advanced slowly. The final amount of shift was large, and the reduction of reflectivity by rinsing, specifically, the reduction of film thickness, was small.

### Example 3

Co-adsorption of nucleic acid probe and MCH, and hybridization 1
(1) The nucleic acid probe, spacer molecule, buffer solution, and solid phase substrate were prepared in the same manner as described in the above-mentioned example 1.
(2) Co-adsorption: Four kinds of mixed solution were prepared by mixing the nucleic acid probe solution and spacer molecule solution (10 µM respectively) prepared in example 1.
   Respective mixed solution contained nucleic acid probe molecule and MCH molecule at a ratio of 25/75, 50/50, 80/20, 90/10 (mol%). A solid phase substrate with clean surfaces was immersed in the mixed solution, and incubated for 1 to 3 hours. SAMs comprising the nucleic acid probe molecule and MCH were formed on the surface of the substrate.
(3) Hybridization reaction: Complementary DNA (target DNA molecule: dT20) was dissolved in PBS buffer solution to make a DNA concentration of 1µM. The DNA solution was brought into contact with the surface of the substrate on which SAMs was formed in (2) above, and a hybridization reaction was induced.
(4) Results: Indicated in Figure 3 are the results of evaluating the degree of adsorption of nucleic acid probe and level of hybridization based on the amount of shift of reflected light in SPR measurements. The change in the intensity of the reflected light is related to the reflectivity shift and the increase or decrease of film thickness. Coverage of the substrate was finally achieved when the nucleic acid probe was at a composition ratio of 90%, or significant hybridization was produced from a composition ratio of 90%. This appears to have been produced because superior adsorption of MCH occurred.

### Example 4

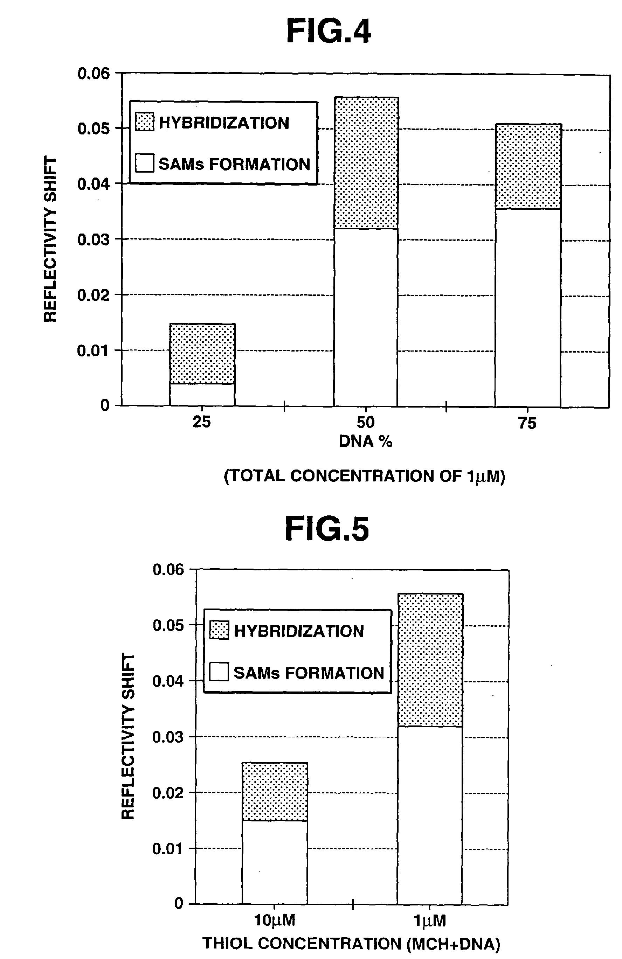

Co-adsorption of nucleic acid probe and MCH, and hybridization 2
(1) The nucleic acid probe, spacer molecule, buffer solution, and solid phase substrate were prepared in the same manner as described in the above-mentioned example 1.
(2) Co-adsorption: Mixed solutions were prepared by mixing the nucleic acid probe solution and spacer molecule solution (1 µM respectively) prepared in example 1.
   Respective mixed solution contained nucleic acid probe molecule and MCH molecule at a ratio of 25/75, 50/50, 75/25 (mol%). A solid phase substrate with clean surfaces was immersed in the mixed solution, and incubated for 1 to 3 hours. SAMs comprising the nucleic acid probe molecule and MCH were formed on the surface of the substrate.
(3) Hybridization reaction: A hybridization reaction was conducted using the same method described in example 3.
(4) Results:
   Indicated in Figure 4 are the results of evaluating the degree of adsorption of the nucleic acid probe and the level of hybridization based on the amount of shift of reflected light in SPR measurements. The change in the strength of the reflected light is related to the reflectivity shift and the increase or decrease of film thickness. At a total concentration of 1 µM, the reflectivity shift caused by hybridization was the greatest at a concentration of roughly 50%. As opposed to the results of subjecting a 10 µM mixed solution to an immobilization reaction, it was extremely easy to control the optimal percentage of the nucleic acid probe.

### Example 5

Solution concentration dependence in co-adsorption and hybridization
(1) The nucleic acid probe, spacer molecule, buffer solution, and solid phase substrate were prepared in the same manner as described in the above-mentioned example 1.
(2) Co-adsorption: Mixed solutions were prepared by mixing the nucleic acid probe solution and spacer molecule solution. 1 µM nucleic acid probe solution is mixed with 1 µM spacer molecule solution, and 10 µM probe solution is mixed with 10 µM spacer molecule solution, at a ratio of 50/50 (mol%). Co-adsorption reactions and hybridization reactions were conducted in conformity to examples 3 and 4, and the reflectivity of the immobilized substrate surface was observed by SPR measurements.
(3) Results:
   Indicated in Figure 5 are the results of evaluating the co-adsorption of the nucleic acid probe and 6-mercapto-1-hexanol, as well as formation of hybridization based on the amount of shift of reflected light in SPR measurements. The change in the strength of the reflected light is related to the reflectivity shift and the increase or decrease of film thickness caused by co-adsorption or hybridization. The concentration dependence of the reflectivity shift is indicated in Figure 5. At a total concentration of 10 µM, it appears that the shift of reflectivity caused by adsorption was small, and that the adsorbed film itself was thin. As a result, the shift based on hybridization was extremely small.

### Example 6

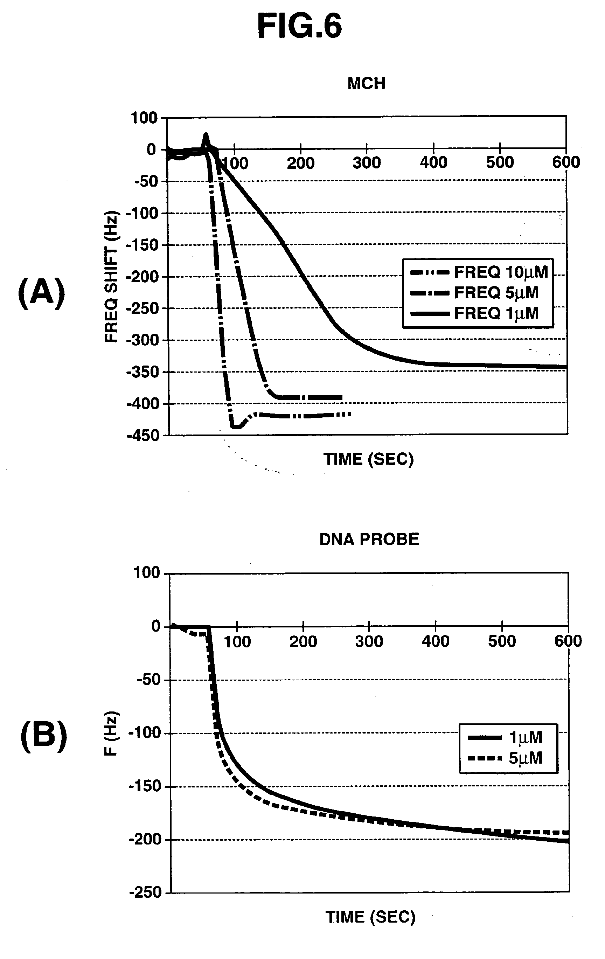

Concentration dependence of DNA probe SAMs formation and 6-mercapto-1-hexanol SAMs formation.
(1) The nucleic acid (DNA) probe, 6-mercapto-1-hexanol, buffer solution, and solid phase substrate were prepared in the same manner as described in the above-mentioned example 1.
(2) SAMs formation test:1 µM and 5 µM of nucleic acid probe solution, and 10 µM, 5 µM and 1 µM of 6-mercapto-1-hexanol solution were prepared. Solid phase substrates were immersed in the respective solutions, and the changes in crystal oscillation frequency were detected by QCM (quartz crystal microbalance).
(3) Results:
   Indicated in Figure 6 are the results of evaluation of SAMs formation of nucleic acid probe and 6-mercapto-1-hexanol films using the amount of frequency change in QCM measurements. The amount of frequency change corresponds to the amount of change of mass number and the increase or decrease in the number of molecules adsorbed. It was demonstrated that 1 µM DNA solution and 1 µM MCH solution had almost equal adsorption speeds.
   From the above results, when the total concentration of a mixed solution comprising nucleic acid probe and MCH was 1 µM, a higher density (i.e., optimal density) of nucleic acid probe was obtained using a comparatively low concentration of nucleic acid probe than when using a total concentration of 10 µM. In addition, the level of hybridization increased thereby, and it became possible to detect greater amount target nucleic acid molecules at one time.

## Claims

1. A method for immobilizing nucleic acid on a solid phase substrate comprising:
bringing the solid phase substrate into contact with a composition comprising a total concentration of 0.1 to 2 µM of a nucleic acid as a probe and a compound or a salt thereof, the compound being represented by the following formula:
HS-L¹-L²-R (I)
wherein L¹ is a single bond or a C₁₋₁₅ alkylene group; L² is a single bond, a nucleic acid, a polyethylene glycol group, -CO-NH-, or -NH-CO-; R is a hydroxyl group, an amino group, a ferrocenyl group, or a carboxyl group; provided that neither L¹ nor L² is a single bond, and
incubating the composition in contact with a surface of the solid phase substrate.

2. The method according to claim 1, wherein the nucleic acid as a probe comprises a polynucleotide or an oligonucleotide consisting of modified or unmodified, single-stranded DNA, RNA, PNA, CNA, or HNA.

3. The method according to claim 1, wherein the nucleic acid as the probe comprises at the 3' end or the 5' end a group represented by the following formula:
HS-L³-L⁴- (II)
wherein L³ is a C₁₋₁₅ alkylene group, and L⁴ is a single bond or a spacer.

4. The method according to claim 1, wherein the nucleic acid as the probe has at the 5' end a group represented by the following formula: 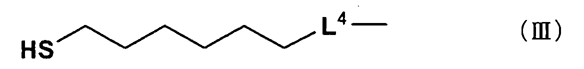 wherein L⁴ is a single bond or a spacer.

5. The method according to claim 4, wherein L⁴ is a nucleic acid, -CO-NH-, -NH-CO-, a polyethylene glycol group, or a polyethylene glycol phosphate group.

6. The method according to claim 1, wherein the total concentration of the nucleic acid and the compound I or the salt thereof in the composition is 0.5 to 1.5 µM.

7. The method according to claim 1, wherein the total concentration of the nucleic acid and the compound I or the salt thereof in the composition is 1 µM.

8. The method according to claim 1, wherein the composition comprises the nucleic acid and the compound I at a ratio of 40/60 to 60/40.

9. The method according to claim 1, wherein R in the formula I is a hydroxyl group.

10. The method according to claim 1, wherein L¹ in the formula I is a single bond, and L² is a polyethylene glycol group.

11. The method according to claim 1, wherein L¹ in the formula I is a C₄₋₈ alkylene group, and L² is a single bond.

12. The method according to claim 1, wherein the compound I is 6-mercapto-1-hexanol.

13. The method according to claim 1, wherein the solid phase substrate is a single layered substrate or a multiple layered substrate comprising at least one material selected from the group consisting of glass, polymer resin and metal.

14. The method according to claim 1, wherein the surface of the solid phase substrate on which nucleic acid is adsorbed is coated with a thin gold film.

15. The method according to claim 1, wherein the solid phase substrate is a glass substrate with a thin gold film vapor-deposited on its surface, and may further comprises, at least one intermediate layer between the thin gold film and the glass substrate.

16. The method according to claim 1, wherein the nucleic acid as the probe has 15 to 30 base length.

17. The method according to claim 1, wherein the incubation is carried out at a temperature of 25° C to 40° C.

18. The method according to claim 1, wherein the nucleic acid as the probe is a polynucleotide or an oligonucleotide consisting of single-stranded DNA, RNA, or PNA, and may also has the group represented by formula II, the compound I is 6-mercapto-1-hexanol; the total concentration of the nucleic acid and 6-mercapto-1-hexanol in the composition is 0.5 to 1.5 µM; and the solid phase substrate is a glass substrate with a thin gold film vapor-deposited on its surface, and further, at least one intermediate layer may be made between the thin gold film and the glass substrate.

19. A method for manufacturing a biosensor having a nucleic acid probe as a sensing site comprising the use of the nucleic acid immobilization method according to any of claims 1 to 18.

20. A biosensor comprising a solid phase substrate and a nucleic acid probe thereon as a sensing site, the biosensor manufactured by a method comprising the steps of:
bringing a composition containing a nucleic acid and a compound or a salt thereof at a total concentration of 0.5 to 1.5 µM into contact with the solid phase substrate, the compound being represented by the following formula:
HS-L¹-L²-R (I)
wherein L¹ is a single bond or a C₁₋₁₅ alkylene group; L² is a single bond, a nucleic acid, a polyethylene glycol group, -CO-NH-, or -NH-CO-; R is a hydroxyl group, an amino group, a ferrocenyl group, or a carboxyl group; provided that neither L¹ nor L² is a single bond, and
incubating the composition in contact with a surface of a solid phase substrate.

21. The biosensor according to claim 20, wherein the compound I is 6-mercapto-1-hexanol, the solid phase substrate is a glass substrate with a thin gold film vapor-deposited on its surface, and further, the solid phase substrate may comprises at least one intermediate layer between the thin gold film and the glass substrate.

22. A method for detecting a target nucleic acid molecule in a test sample by detecting a measurable signal, comprising the steps of:
(a) bringing the test sample into contact with a biosensor having a nucleic acid probe manufactured by using the nucleic acid immobilization method according to claim 1, and
incubating the test sample in contact with a surface of the biosensor;
(b) applying light to the solid phase substrate of the biosensor from the opposite side of the surface to which nucleic acid is immobilized, continuously or intermittently from before to after step (a); and
(c) measuring a shift of reflectivity of the solid phase substrate by detecting the reflection of the light applied in step (b).

23. The method according to claim 22, wherein the method is used for detecting DNA, RNA, or single nucleotide polymorphisms.

24. A use of a composition comprising a nucleic acid and compound I or salt thereof at a total concentration of 0.5 to 1.5 µM, for manufacturing a biosensor having a nucleic acid probe as a sensing site.

25. A use of a nucleic acid and compound I or salt thereof, for manufacturing the composition for the use according to claim 24.
